**Europäisches Patentamt**

**European Patent Office**

**Office européen des brevets**

(11) Numéro de publication: **0 337 901**
**A1**

## (12) DEMANDE DE BREVET EUROPEEN

(21) Numéro de dépôt: 89420118.5

(22) Date de dépôt: 31.03.89

(51) Int. Cl.⁴: **A 61 B 17/14**
A 61 F 2/46

(30) Priorité: 01.04.88 FR 8804782

(43) Date de publication de la demande:
18.10.89 Bulletin 89/42

(84) Etats contractants désignés:
AT BE CH DE ES GB GR IT LI LU NL SE

(71) Demandeur: **Broc, Christian**
**Les Beaumettes**
**F-84220 Gordes (FR)**

(72) Inventeur: **Broc, Christian**
**Les Beaumettes**
**F-84220 Gordes (FR)**

(74) Mandataire: **Dupuis, François**
**Cabinet Charras 3 Place de l'Hôtel-de-Ville BP 203**
**F-42005 St. Etienne Cédex 1 (FR)**

(54) **Matériel de pose pour notamment un élément tibial et/ou fémoral d'une prothèse bi-compartimentaire d'articulation du genou.**

(57) Le matériel de pose est remarquable en ce que le support (1) reçoit à libre coulissement avec capacité de blocage en position, une tige (2) en bout de laquelle est fixée un guide profilé courbe (4) pour correspondre au profil du tibia, ledit guide (4) formant un plan d'appui horizontal, ladite tige (2) étant agencée pour être fixée temporairement sur une partie du tibia.

FIG.1

EP 0 337 901 A1

Bundesdruckerei Berlin

## Description

## Matériel de pose pour notamment un élément tibial et/ou fémoral d'une prothèse bi-compartimentaire d'articulation du genou.

L'invention concerne plus particulièrement un matériel ou instrumentation de pose des implants, d'une prothèse bi-compartimentaire du genou comprenant des implants fémoraux du type de ceux dont le profil en section correspond au rayon de courbure moyen du condyle et des implants tibiaux du type de ceux formés avec un patin ou plateau d'appui apte à coopérer avec la partie reséquée du massif des épines tibiales.

Les matériels employés à ce jour ne donnent pas entièrement satisfaction.

En ce qui concerne la mise en place de l'implant tibial, il est connu d'utiliser, après avoir pris bien évidemment, les différentes radiographies et mesures nécessaires, un appareil dénommé viseur tibial qui comprend un support destiné à être positionné sur la crête de l'axe général de la diaphyse du tibia. Dans ce support est monté coulissant le viseur proprement dit qui occupe un volume important de sorte que sur un genou ouvert, il apparaît des problèmes importants de mise en place d'un tel appareil.

Un autre problème très important réside dans le fait que le viseur, destiné à recevoir une lame de scie ou autre doit être maintenu en position manuellement, généralement par un assistant du praticien. On conçoit que cette façon d'opérer est peu rationnelle et très aléatoire risquant de nuire à la qualité et au résultat de pose de l'implant.

Pour remédier à ces inconvénients et résoudre les problèmes posés, selon l'invention, le support du viseur tibial reçoit à libre coulissement avec capacité de blocage en position, une tige en bout de laquelle est fixé un guide profilé courbe pour correspondre au profil du tibia, ledit guide formant un plan d'appui horizontal, ladite tige étant agencée pour être fixée temporairement sur une partie du tibia.

En outre, un autre problème que se propose de résoudre l'invention est de pouvoir respecter au maximum le plan de coupe tibial, initialement déterminé, ce qui n'est pas permis avec les moyens employés actuellement qui obligent d'opérer d'une manière approximative et par étapes successives.

Ce problème est résolu selon l'invention, en ce que le guide tibial présente une fente pour le passage d'organes du type broches aptes à coopérer avec le tibia pour indiquer et conserver le plan de coupe tibial initialement calculé, ladite broche étant susceptible de recevoir, après enlèvement de l'ensemble du guide tibial, un gabarit de coupe réversible.

On a voulu également pallier aux éventuelles erreurs faites au niveau du plan de coupe pour tenir compte des différences qui peuvent exister entre la partie frontale et la partie postérieure. Un tel problème est résolu selon l'invention compte-tenu du fait que le gabarit de coupe est engagé sur les broches au moyen d'une fente disposée d'une manière asymétrique dans l'épaisseur dudit gabarit tout en étant en correspondance, d'un côté, avec la fente du gabarit tibial.

Il apparaît également que dans le matériel ancillaire de pose, il n'est pas tenu compte des composantes rotatoires du genou, c'est-à-dire de la coupe frontale du massif des épines spirales.

Pour résoudre ce problème selon l'invention, le gabarit de coupe reçoit dans un plan perpendiculaire un plot d'appui vertical coopérant avec une broche convenablement orientée pour indiquer l'angle de coupe du massif des épines tibiales en fonction de la rotation du genou, ledit plot constitue un moyen de guidage et d'appui à l'organe de coupe.

En ce qui concerne la pose de l'implant fémoral pour tenir compte des contraintes rotatoires au moment de la flexion du genou, il est nécessaire que le positionnement de l'implant soit parfait sur les trois plans de l'espace. Le plan de courbure frontale doit donc regarder la charge.

Pour résoudre ce problème et atteindre le but recherché, selon l'invention, on utilise un guide-coupe fémoral postérieur composé d'une tige recevant en bout un patin d'appui courbe dont le rayon de courbure correspond au patin condylien qui est remarquable en ce que la tige est positionnée, par rapport au patin d'appui, de manière a être disposée en correspondance et en alignement axial avec le plot d'ancrage que présente normalement l'implant tibial devant être posé, ladite tige étant percée axialement pour permettre l'engagement d'une mèche.

L'invention est exposée ci-après plus en détail à l'aide des dessins annexés, dans lesquels :

- la figure 1 est une vue en perspective montrant les principaux éléments de matériel de pose pour les implants tibiaux,
- les figures 2, 3, 4, 5 et 6 sont des vues à caractère purement schématique montrant les principales phases opératoires pour la préparation du compartiment tibial,
- les figures 7 et 8 sont des vues en coupe respectivement du guide tibial et du gabarit de coupe tibial,
- la figure 9 est une vue en perspective du guide de coupe fémoral,
- la figure 10 est une vue en coupe longitudinale du guide-coupe fémoral, mis en place sur le condyle en position de flexion du genou, en combinaison avec le plan de coupe tibiale,
- la figure 11 est une vue en plan, à caractère schématique montrant le positionnement du guide fémoral.

Afin de rendre plus concret l'objet de l'invention, on le décrit maintenant d'une manière non limitative en se référant aux exemples de réalisation des figures des dessins.

Le viseur de coupe tibial comprend un support (1) dont la section transversale est profilée en V sur la totalité de sa hauteur, pour être appliqué, positionné et centré en combinaison avec l'axe général de la diaphyse du tibia. Ce type de support est connu.

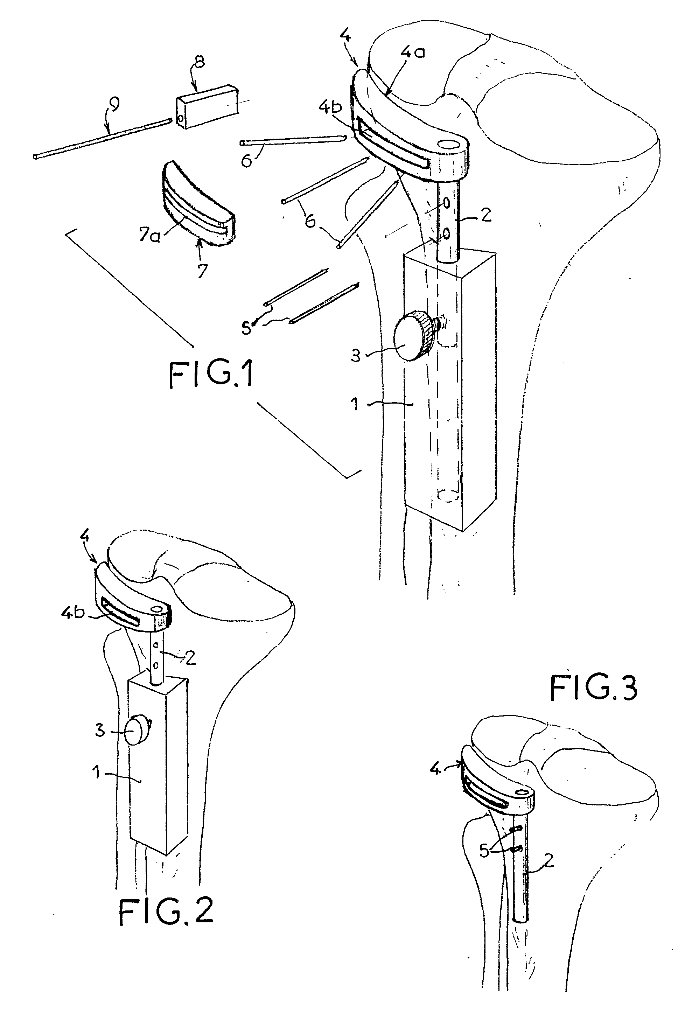

Selon l'invention, le support (1) reçoit à libre coulissement une tige (2) montée avec capacité de blocage en position verticale selon différentes hauteurs. Par exemple, le blocage peut être effectué au moyen d'une vis (3) engagée dans une lumière oblongue (2a) de la tige et vissée dans le support (1). Cette tige (2) reçoit en bout, notamment d'une manière démontable, un guide profilé (4) formant un plan d'appui horizontal. Le guide (4) présente un rayon de courbure interne (4a) correspondant très sensiblement au profil interne ou externe du tibia. Il y a donc deux guides (4) pour correspondre au profil droit et au profil gauche, selon le cas (figure 2).

La partie supérieure de la tige (2) qui apparaît en débordement du support (1), présente des trous pour l'engagement de broches de fixation (5) en vue d'assurer le maintien en position de l'ensemble du guide (4) et de la tige (2). Dans ces conditions, le support (1) peut être enlevé (figure 3).

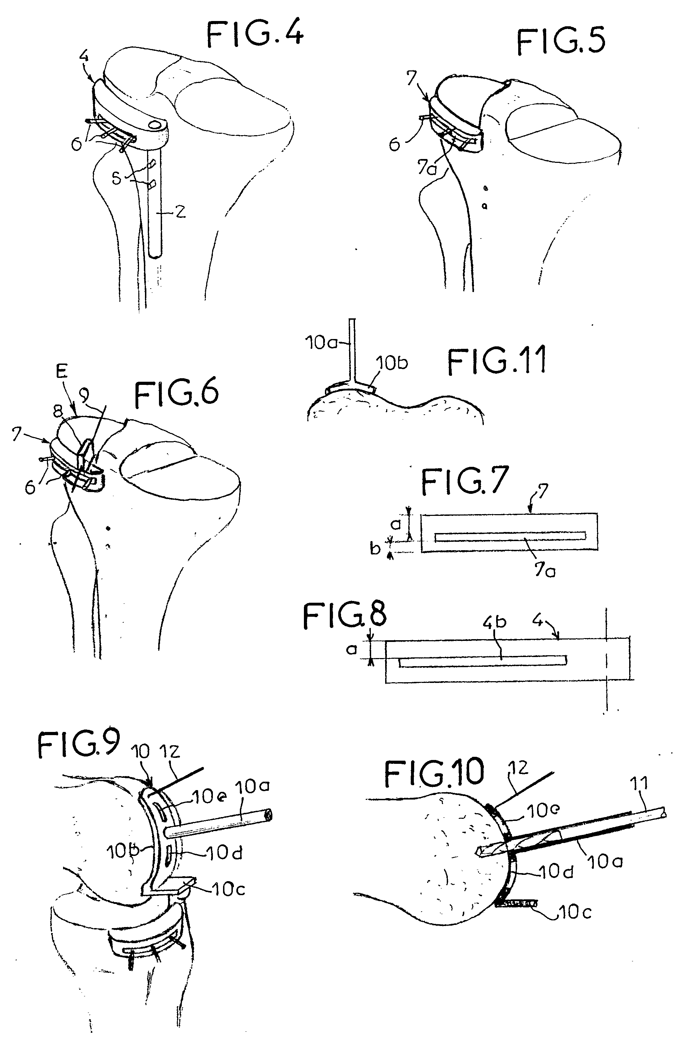

En outre, le guide tibial (4) présente, dans un plan horizontal, une fente longitudinale (4b) au travers de laquelle peuvent être engagées des broches (6). Ces broches (6), notamment au nombre de trois, ont pour fonction essentielle de maintenir et d'indiquer le plan de coupe tibiale initialement calculée, d'une manière classique, après les différentes radiographies et mesures effectuées. En effet, après la mise en place de ces broches (6) (figure 4), il est possible d'enlever l'ensemble guide tibial (4) et tige (2), le niveau du plan de coupe à effectuer étant toujours indiqué par lesdites broches.

Ces broches (6) font office de support à un gabarit de coupe (7) de dimension réduite (figure 5). Dans ce but, le gabarit (7) présente dans un plan horizontal, une fente (7a) apte à coopérer avec l'ensemble des broches.

D'une manière importante, la fente (7a) est établie d'une manière asymétrique par rapport à l'épaisseur du gabarit (7) pour le rendre réversible et utilisable selon deux plans horizontaux différents.

Cette fente (7a) est établie à une distance (a) de l'un de ses bords et à une distance (b) par rapport à son autre bord, la distance (a) étant supérieure à la distance (b). En outre, la distance (a) correspond très exactement à la distance (a') séparant la fente (4b) du bord du guide tibial (4). Il apparaît donc qu'après positionnement du gabarit de coupe (7) sur les broches (6) d'une manière identique au guide tibial (4), (selon la distance (a)), si la coupe effectuée n'est pas suffisante, il suffit de retourner le gabarit pour changer en conséquence le niveau de la coupe tibiale.

En ce qui concerne le problème de l'appréciation de la rotation du genou, c'est-à-dire de la coupe frontale du massif des épines spirales, selon une certaine orientation angulaire, on utilise un plot d'appui vertical (8). Ce plot (8) est agencé pour coopérer avec une broche de positionnement (9) destinée à indiquer l'angle de coupe ( ) du massif des épines Le positionnement angulaire de cette broche (9) a été illustré et décrit dans la demande de brevet 87.06043, dont le demandeur de la présente est également titulaire. Succintement, on rappelle que cette broche coopère avec le bord interne du condyle externe (E) à 90 degrés de flexion du genou pour indiquer la coupe angulaire à effectuer sur le côté correspondant du massif des épines tibiales en fonction de la rotation du genou.

Il suffit dans ces conditions, d'engager le plot (8) sur la broche (9), en appui vertical sur le gabarit de coupe (7) dans un plan perpendiculaire (figure 6). Le plot (8) fait office de contre-appui de guidage à l'organe de coupe.

Au niveau du fémur, le matériel utilisé comprend un guide de coupe fémoral désigné dans son ensemble par (10).

Le guide de coupe comprend une tige (10a) solidaire d'un patin d'appui courbe (10b), dont le rayon de courbure est déterminé pour correspondre très sensiblement au profil des condyles.

D'une manière importante, la tige (10a) est disposée par rapport au patin d'appui-courbe (10b) de manière à être positionnée en correspondance et en alignement axial avec le plot d'ancrage que présente normalement l'implant tibial devant être posé.

Cette tige (10a) dont la longueur est d'au moins 10 cm, et très importante et permet de positionner très exactement dans les conditions bio-mécaniques optimum, le patin d'appui courbe (10b), notamment au milieu du plateau tibial correspondant. La tige (10a) est creuse pour permettre le passage d'une mêche (11) destinée à masquer l'implantation du plot d'ancrage.

En outre, il est nécessaire de tenir compte des contraintes rotatoires au moment de la flexion du genou de sorte que le positionnement du futur implant doit être parfait dans les trois plans de l'espace en respectant la courbure condylienne, l'asymétrie du condyle et en considérant que le plan de courbure frontale doit regarder la charge dynamique et/ou statique (figure 11). En conséquence, la tige permet de bien visualiser la façon dont il faut raboter de manière à avoir immédiatement la bonne orientation pour l'implant et indique de combien il faut meuler le condyle pour avoir un bon plaquage sur le condyle en fonction de l'axe de charge que l'on veut lui donner.

En flexion, il est également nécessaire de positionner l'implant rigoureusement perpendiculaire au plan de coupe tibiale. A cet effet l'extrémité inférieure du patin courbe d'appui (10b) présente un rebord équerré d'appui (10c) pour vérifier que le plan de coupe condylienne postérieur est parfaitement parallèle au plan de coupe tibial pour éviter tout porte à faux et charge supplémentaire (figure 9)

Le patin courbe d'appui (10b) présente, de part et d'autre de la tige (10a) et selon son axe médian, deux fentes (10d) et (10e) pour marquer, au moyen d'un bistouri par exemple, le positionnement angulaire déterminé. Il suffit alors de mettre en correspondance l'implant définitif avec ces lignes de marquage, le plot d'ancrage dudit implant étant mis en regard avec l'emplacement du trou déterminé par la tige creuse (10a).

A noter que l'on prévoit, pour assurer le positionnement anti-rotatoire de l'implant (10), de fixer dans la partie supérieure du patin d'appui (10b) une broche (12).

**Revendications**

- 1 - Matériel de pose pour un élément tibial et fémoral d'une prothèse bi-compartimentaire d'articulation du genou, caractérisé en ce qu'il comprend un support (1) apte à être postionné en combinaison avec l'axe général de la diaphyse du tibia, ledit support (1) reçevant à libre coulissement avec capacité de blocage en position, une tige (2) en bout de laquelle est fixé un guide profilé courbe (4) pour correspondre au profil du tibia, ledit guide (4) formant un plan d'appui horizontal, ladite tige étant agencée pour être fixée temporairement sur une partie du tibia.

- 2 - Matériel selon la revendication 1, caractérisé en ce que le guide tibial (4) présente une fente (4b) pour le passage d'organes du type broches (6) aptes à coopérer avec le tibia pour indiquer et conserver le plan de coupe tibial initialement calculé, lesdites broches (6) étant susceptibles de recevoir, après enlèvement de l'ensemble du guide tibial, un gabarit de coupe réversible (7).

- 3 - Matériel selon la revendication 2, caractérisé en ce que le gabarit de coupe (7) est engagé sur les broches (6) au moyen d'une fente (7a) disposée d'une manière asymétrique dans l'épaisseur dudit gabarit tout en étant en correspondance, d'un côté, avec la fente du gabarit tibial (4).

- 4 - Matériel selon la revendication 3, caractérisé en ce que le gabarit de coupe (4) reçoit dans un plan perpendiculaire, un plot d'appui vertical (8) coopérant avec une broche (9) convenablement orientée pour indiquer l'angle de coupe du massif des épines tibiales en fonction de la rotation du genou, ledit plot (8) constitue un moyen de guidage et d'appui à l'organe de coupe.

- 5 - Matériel selon la revendication 1, caractérisé en ce qu'il comprend un guide de coupe fémoral postérieur (10) composé d'une tige (10a) recevant en bout un patin d'appui courbe (10b) dont le rayon de courbure correspond au patin condylien, ladite tige (10a) étant positionnée, par rapport au patin d'appui (10b) de manière à être disposée en correspondance et en alignement axial avec le plot d'ancrage que présente normalement l'implant tibial devant y être posé, ladite tige (10a) étant percée axialement pour permettre l'engagement d'une mêche.

- 6 - Matériel selon la revendication 5, caractérisé en ce que l'extrémité inférieure du patin d'appui courbe (10b), présente un rebord équerré d'appui (10c) pour vérifier que le plan de coupe condylienne postérieur est parallèle au plan de coupe tibial.

- 7 - Matériel selon l'une quelconque des revendications 5 et 6, caractérisé en ce que le patin courbe d'appui (10b) présente, de part et d'autre de la tige (10a) et selon son axe médian, des moyens (10d-10e) aptes à indiquer le positionnement angulaire déterminé.

FIG.1

FIG.2

FIG.3

EP 0 337 901 A1

FIG.4

FIG.5

4

6

5

2

7

6

7a

E

8 9

7

6

FIG.6

10a

10b

FIG.11

FIG.7

7

a
b

7a

FIG.8

a

4b 4

FIG.9

10 12

10e 10a

10b

10d

10c

FIG.10

12

10e

11

10d 10a

10c

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | US-A-4 524 766  (PETERSEN) <br> * revendications 1-3; figures 2,7,10 * | 1 | A 61 B   17/14 <br> A 61 F    2/46 |
| A |  | 5 | |
| Y | EP-A-0 104 732  (QUEENS UNIVERSITY AT KINGSTON) <br> * revendications 1,2; figures 1,5 * | 1 | |
| Y | US-A-4 566 448  (ROHR) <br> * figure 1 * | 1 | |
| A | US-A-4 457 307  (STILLWELL) <br> * revendication 1; figures 1,5-10 * | 1,5 | |
| A | US-A-4 574 794  (COOKE et al.) <br> * figure 1 * | 1,5 | |
| A | EP-A-0 095 296  (MINNESOTA MINING AND MANUFACTURING COMPANY) <br> * revendication 1 * | 1 | |

---

**DOMAINES TECHNIQUES RECHERCHES (Int. Cl.5)**

A 61 B   17/00
A 61 F    2/00

**Le présent rapport a été établi pour toutes les revendications**

| Lieu de la recherche | Date d'achèvement de la recherche | Examinateur |
|---|---|---|
| BERLIN | 20-06-1989 | KANAL P K |

EPO FORM 1503 03.82 (P0402)